(19) Europäisches Patentamt

European Patent Office

Office européen des brevets

(11) **EP 3 320 939 A1**

(12) **EUROPEAN PATENT APPLICATION**
published in accordance with Art. 153(4) EPC

(43) Date of publication:
**16.05.2018 Bulletin 2018/20**

(21) Application number: **16823696.6**

(22) Date of filing: **15.04.2016**

(51) Int Cl.:
***A61M 16/00*** (2006.01)

(86) International application number:
**PCT/CN2016/079343**

(87) International publication number:
**WO 2017/008549 (19.01.2017 Gazette 2017/03)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **14.07.2015 CN 201510413381**

(71) Applicant: **Beijing Aeonmed Co., Ltd.
Beijing 100070 (CN)**

(72) Inventor: **CHENG, Jie
Beijing 100070 (CN)**

(74) Representative: **Hanna Moore + Curley
Garryard House
25/26 Earlsfort Terrace
Dublin 2, D02 PX51 (IE)**

(54) **CLOSED LOOP CAPACITY CONTROL METHOD FOR RESPIRATOR**

(57) The present invention provides a closed-loop volume control method of a breathing machine, comprising: firstly generating a voltage-flow velocity curve of a proportional valve of the breathing machine, and calculating a set inspiratory flow velocity of the breathing machine according to an actual volume VT and an inspiratory time T of the breathing machine; and letting an initial control voltage as a voltage corresponding to the set inspiratory flow velocity in the voltage-flow velocity curve, and time-division calculating the voltage controlling the inspiratory flow velocity by a closed-loop control algorithm within the inspiratory time until a real-time inspiratory flow velocity reaches a requirement of the set inspiratory flow velocity under the control voltage. The method of the invention possesses a feature of a high precision in controlling a flow velocity, as well as is quick in adjustment, without an overshot generated flow velocity and a misadjustment in control.

generating a voltage-flow velocity curve of a proportional valve of a breathing machine

↓

calculating a set inspiratory flow velocity of the breathing machine according to an actual volume VT and an inspiratory time T of the breathing machine

↓

letting an initial control voltage as a voltage corresponding to the set inspiratory flow velocity in the voltage-flow velocity curve, and time-division calculating the voltage controlling the inspiratory flow velocity by a closed-loop control algorithm until a real-time inspiratory flow velocity reaches a requirement of the set inspiratory flow velocity under the control voltage

↓

enabling the breathing machine to enter a stable operating status; acquiring a final control voltage in step 3), and controlling the proportional valve of the breathing machine with the final control voltage to cause the flow velocity to meet a set requirement

Fig. 2

EP 3 320 939 A1

**Description**

**TECHNICAL FIELD**

The present invention relates to the field of a breathing machine, in particular to a closed-loop volume control method of the breathing machine.

**BACKGROUND OF THE INVENTION**

A volume control (VCV) mode is the most traditional control mode for a breathing machine. In the early development of the breathing machine, the VCV control model is always dominative, with each category of breathing machines having a VCV model. However, there is a different control algorithm for each type of breathing machines.
Currently, some breathing machines adopt an open-loop control manner due to a defective volume control method, resulting in an actually provided tidal volume far deviation from a set value, which will cause harm to users of the breathing machines, and therefore pose a potential risk.

**SUMMARY OF THE INVENTION**

The present invention aims to overcome the above-described defect existing in the current volume control method of a breathing machine, proposing a closed-loop volume control method of a breathing machine. The method has features of a high control precision, a quick adjustment time, no overshoot of a flow velocity and no misadjustment in control.
In order to achieve the above-described objective, the present invention provides a closed-loop volume control method of a breathing machine, comprising: firstly generating a voltage-flow velocity curve of a proportional valve of the breathing machine, and calculating a set inspiratory flow velocity of the breathing machine according to an actual volume VT and an inspiratory time T of the breathing machine; and letting an initial control voltage as a voltage corresponding to the set inspiratory flow velocity in the voltage-flow velocity curve, and then time-division calculating the voltage controlling the inspiratory flow velocity by a closed-loop control algorithm until a real-time inspiratory flow velocity reaches a requirement of the set inspiratory flow velocity under the control voltage.
In the above technical solution, the method specifically comprises:

step 1) generating the voltage-flow velocity curve of the proportional valve of the breathing machine;
step 2) calculating the set inspiratory flow velocity of the breathing machine according to the actual volume VT and the inspiratory time T of the breathing machine:

$$V = VT/T$$

wherein VT is the actual volume, T is the inspiratory time, and V is the inspiratory flow velocity with a unit of ml/s;
step 3) letting the initial control voltage as the voltage corresponding to the set inspiratory flow velocity in the voltage-flow velocity curve, time-division calculating the voltage controlling the inspiratory flow velocity by a closed-loop control algorithm within the inspiratory time until a real-time inspiratory flow velocity reaches the requirement of the set inspiratory flow velocity under the control voltage; and
step 4) enabling the breathing machine to enter a stable operating status; acquiring a final control voltage in step 3), and controlling the proportional valve of the breathing machine with the final control voltage to cause the flow velocity to meet a set requirement.

In the above technical solution, a specific implementing process of the step 1) is: collecting a voltage and a value of a corresponding flow velocity of the proportional valve of the breathing machine every 0.2 ms while the breathing machine operates normally, and performing a linear fitting using the least square method to generate the voltage-flow velocity curve of the proportional valve of the breathing machine.
In the above technical solution, the step 3) specifically comprises:

step 301) obtaining the voltage corresponding to the set inspiratory flow velocity V using the voltage-flow velocity curve generated in the step 1), wherein a value of the voltage is a value of a control voltage $P_0$ at an initial time $t_0$; and let k = 1;
step 302) controlling the proportional valve with the value of the control voltage $P_{k-1}$, and measuring a flow velocity corresponding to time $t_{k-1}$ by a sensor as $V_{k-1}$; and

step 303) calculating the value of the control voltage $P_k$ at time $t_k$:

$$up[k] = kp \times (V - V_{k-1}) + P_{k-1}$$

$$ui[k] = ki \times (V - V_{k-1})$$

$$P_k = up[k] + ui[k]$$

wherein V is the set velocity, $V_{k-1}$ is the flow velocity measured by the sensor at time $t_{k-1}$, and $P_{k-1}$ is the control voltage at time $t_{k-1}$, i.e., a feedforward voltage; and kp is a proportional coefficient, ki is an integral coefficient, and up[k] and ui[k] are intermediate values;

step 304) controlling the proportional valve with the control voltage $P_k$ at time $t_k$, and measuring the flow velocity $V_k$ at time $V_k$ by the sensor; and

step 305) determining whether an absolute value of a difference between $V_k$ and the set flow velocity V is less than a threshold, and if a determining result is affirmative, proceeding to the step 4); otherwise, letting k = k + 1, and proceeding to the step 303).

In the above technical solution, the threshold is 2 ml/s.

In the above technical solution, the proportional coefficient of the step 303) is 0.2.

In the above technical solution, the integral coefficient of the step 303) is 0.005.

The advantages of the present invention lie in that:

1. the method of the present invention has a feature of a high precision in controlling a flow velocity; and
2. the method of the present invention is quick in adjustment, without an overshot generated flow velocity and a misadjustment in control.

## BRIEF DESCRIPTION OF THE DRAWINGS

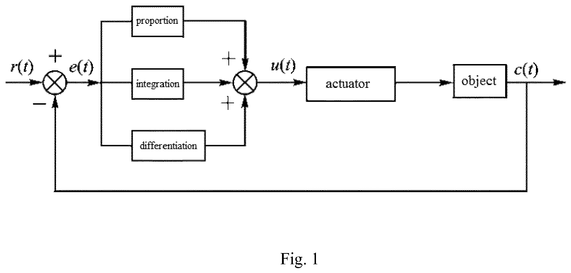

Fig. 1 is a block diagram of principles of an analog PID control system; and
Fig. 2 is a flow chart of a closed-loop volume control method of a breathing machine of the present invention.

## DETAILED DESCRIPTION OF THE INVENTION

The present invention is further described in detail below in conjunction with the drawings and specific embodiments.

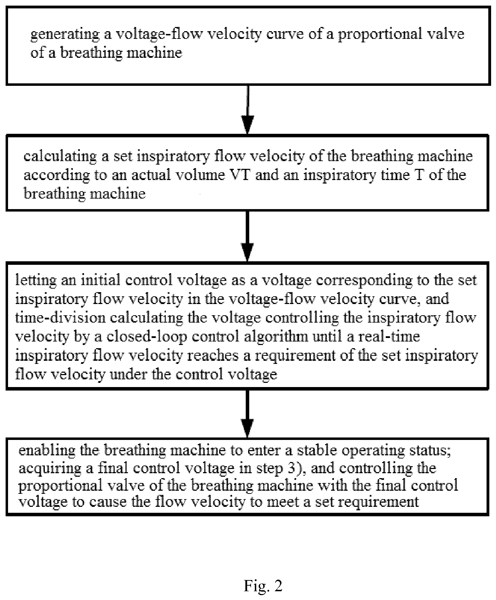

As shown in Fig. 2, a closed-loop volume control method of a breathing machine comprises:

step 1) generating a voltage-flow velocity curve of a proportional valve of the breathing machine;
a voltage and a value of a corresponding flow velocity of the proportional valve of the breathing machine every 0.2 ms are collected while the breathing machine operates normally, and a linear fitting is performed using the least square method to generate the voltage-flow velocity curve of the proportional valve of the breathing machine.
Step 2) calculating a set inspiratory flow velocity of the breathing machine according to an actual volume VT and an inspiratory time T of the breathing machine:

$$V = VT/T$$

wherein VT is the actual volume, T is the inspiratory time, and V is the inspiratory flow velocity with a unit of ml/s; volume control is to ensure that the monitored flow velocity equals to the set flow velocity during the whole inspiratory process, in this way the actually provided inspiratory tidal volume is ensured to be in close proximity to the set value VT.

Step 3) letting an initial control voltage as a voltage corresponding to the set inspiratory flow velocity in the voltage-flow velocity curve, time-division calculating the voltage controlling the inspiratory flow velocity by a closed-loop control algorithm within the inspiratory time until a real-time inspiratory flow velocity reaches a requirement of the set inspiratory flow velocity under the control voltage, the step 3) specifically comprising:

step 301) obtaining the voltage corresponding to the set inspiratory flow velocity V using the voltage-flow velocity curve generated in the step 1), wherein a value of the voltage is a value of a control voltage $P_0$ at an initial time $t_0$; and let $k = 1$;

step 302) controlling the proportional valve with the value of the control voltage $P_{k-1}$, and measuring a flow velocity corresponding to time $t_{k-1}$ by a sensor as $V_{k-1}$;

preferably, $t_k - t_{k-1} = 0.03s$;

step 303) calculating the value of the control voltage $P_k$ at time $t_k$:

$$up[k] = kp \times (V - V_{k-1}) + P_{k-1}$$

$$ui[k] = ki \times (V - V_{k-1})$$

$$P_k = up[k] + ui[k]$$

wherein V is the set flow velocity, $V_{k-1}$ is the flow velocity measured by the sensor at time $t_{k-1}$, and $P_{k-1}$ is the control voltage at time $t_{k-1}$, i.e., a feedforward voltage; and kp is a proportional coefficient with a preferential value of 0.2, ki is an integral coefficient with a preferential value of 0.005; and up[k] and ui[k] are intermediate values;

step 304) controlling the proportional valve with the control voltage $P_k$ at time $t_k$, and measuring the flow velocity $V_k$ at time $V_k$ by the sensor; and

step 305) determining whether an absolute value of a difference between $V_k$ and the set flow velocity V is less than a threshold, and if a determining result is affirmative, proceeding to the step 4); otherwise, letting $k = k + 1$, and proceeding to the step 303);

preferably, the threshold is 2 ml/s; and

step 4) enabling the breathing machine to enter a stable operating status; calculating the control voltage with the step 303), and using the control voltage, then the flow velocity of the proportional valve of the breathing machine meets a set requirement.

Example 1:

Four groups of parameters were set on the breathing machine, wherein an inspiratory time was 1 s, and volumes were set as 100 ml, 200 ml, 300 ml and 2000 ml, respectively. Monitored tidal volumes of the breath machine VT1s and VT2s of a detecting device were recorded, respectively. The control precision and the measuring precision were all within an error range. Specific data are seen in Table 1:

Table 1

| Set value | Monitored value of breathing machine VT1 | Monitored value of detecting device VT2 |
|---|---|---|
| 100 | 98 | 102 |
| 200 | 202 | 210 |
| 300 | 298 | 308 |
| 2000 | 1980 | 2010 |

From the above results, it can be seen that with the method of the present invention a high control precision and quick adjustment speed are achieved. When the tidal volume is adjusted from 100 to 200, the tidal volume monitored in the next cycle VTI=202. However, with a traditional VCV, a method of adjusting once every cycle is employed, then the adjustment is quite slow. For example, if a tidal volume is set as 400, a monitored value in a first cycle is 300, which will be adjusted upward slowly in a next cycle, for example, to 330, and therefore needs several cycles to be controlled to a required accuracy. In contrast, the method of the present invention controls once every 3 ms, therefore a flow velocity can be adjusted to a set flow velocity within a short time. Generally, one cycle suffices to achieve a control objective.

**Claims**

1. A closed-loop volume control method of a breathing machine, comprising: firstly generating a voltage-flow velocity curve of a proportional valve of the breathing machine, and calculating a set inspiratory flow velocity of the breathing machine according to an actual volume VT and an inspiratory time T of the breathing machine; and letting an initial control voltage as a voltage corresponding to the set inspiratory flow velocity in the voltage-flow velocity curve, and time-division calculating the voltage controlling the inspiratory flow velocity by a closed-loop control algorithm within the inspiratory time until a real-time inspiratory flow velocity reaches a requirement of the set inspiratory flow velocity under the control voltage.

2. The closed-loop volume control method of a breathing machine according to claim 1, wherein the method specifically comprises:

   step 1) generating the voltage-flow velocity curve of the proportional valve of the breathing machine;
   step 2) calculating the set inspiratory flow velocity according to the actual volume VT and the inspiratory time T of the breathing machine:

   $$V=VT/T$$

   wherein VT is the actual volume, T is the inspiratory time, and V is the inspiratory flow velocity with a unit of ml/s;
   step 3) letting the initial control voltage as the voltage corresponding to the set inspiratory flow velocity in the voltage-flow velocity curve, time-division calculating the voltage controlling the inspiratory flow velocity by a closed-loop control algorithm within the inspiratory time until a real-time inspiratory flow velocity reaches the requirement of the set inspiratory flow velocity under the control voltage; and
   step 4) enabling the breathing machine to enter a stable operating status; acquiring a final control voltage in step 3), and controlling the proportional valve of the breathing machine with the final control voltage to cause the flow velocity to meet a set requirement.

3. The closed-loop volume control method of a breathing machine according to claim 2, wherein a specific implementing process of the step 1) is: collecting a voltage and a value of a corresponding flow velocity of the proportional valve of the breathing machine every 0.2 ms while the breathing machine operates normally, and performing a linear fitting using the least square method to generate the voltage-flow velocity curve of the proportional valve of the breathing machine.

4. The closed-loop volume control method of a breathing machine according to claim 2, wherein the step 3) specifically comprises:

   step 301) obtaining the voltage corresponding to the set inspiratory flow velocity V using the voltage-flow velocity curve generated in the step 1), wherein a value of the voltage is a value of control voltage $P_0$ at an initial time $t_0$; and let $k = 1$;
   step 302) controlling the proportional valve with the value of the control voltage $P_{k-1}$, and measuring a flow velocity corresponding to time $t_{k-1}$ by a sensor as $V_{k-1}$; and
   step 303) calculating the value of the control voltage $P_k$ at time $t_k$:

   $$up[k] = kp \times (V - V_{k-1}) + P_{k-1}$$

   $$ui[k] = ki \times (V - V_{k-1})$$

   $$P_k = up[k] + ui[k]$$

   wherein V is the set flow velocity, $V_{k-1}$ is the flow velocity measured by the sensor at time $t_{k-1}$, and $P_{k-1}$ is the control voltage at time $t_{k-1}$, i.e., a feedforward voltage; and kp is a proportional coefficient, ki is an integral coefficient, and $up[k]$ and $ui[k]$ are intermediate values;

step 304) controlling the proportional valve with the control voltage $P_k$ at time $t_k$, and measuring the flow velocity $V_k$ at time $V_k$ by the sensor; and

step 305) determining whether an absolute value of a difference between $V_k$ and the set flow velocity V is less than a threshold, and if a determining result is affirmative, proceeding to the step 4); otherwise, letting k = k + 1, and proceeding to the step 303).

5. The closed-loop volume control method of a breathing machine according to claim 4, wherein the threshold is 2 ml/s.

6. The closed-loop volume control method of a breathing machine according to claim 4, wherein the proportional coefficient of the step 303) is 0.2.

7. The closed-loop volume control method of a breathing machine according to claim 4, wherein the integral coefficient of the step 303) is 0.005.

Fig. 1

generating a voltage-flow velocity curve of a proportional valve of a breathing machine

calculating a set inspiratory flow velocity of the breathing machine according to an actual volume VT and an inspiratory time T of the breathing machine

letting an initial control voltage as a voltage corresponding to the set inspiratory flow velocity in the voltage-flow velocity curve, and time-division calculating the voltage controlling the inspiratory flow velocity by a closed-loop control algorithm until a real-time inspiratory flow velocity reaches a requirement of the set inspiratory flow velocity under the control voltage

enabling the breathing machine to enter a stable operating status; acquiring a final control voltage in step 3), and controlling the proportional valve of the breathing machine with the final control voltage to cause the flow velocity to meet a set requirement

Fig. 2

# INTERNATIONAL SEARCH REPORT

| International application No. |
| --- |
| **PCT/CN2016/079343** |

### A. CLASSIFICATION OF SUBJECT MATTER

A61M 16/00 (2006.01) i

According to International Patent Classification (IPC) or to both national classification and IPC

### B. FIELDS SEARCHED

Minimum documentation searched (classification system followed by classification symbols)

A61M 16/-;

Documentation searched other than minimum documentation to the extent that such documents are included in the fields searched

Electronic data base consulted during the international search (name of data base and, where practicable, search terms used)

CNPAT, WPI, EPODOC, CNKI: BEIJING AEONMED CO., LTD.; CHENG, Jie; closed loop, volume control, humidity, actual, meter, proportional valve, current, curve, flow, ventilator; loop, volume, control, velocity, valve, breath+

### C. DOCUMENTS CONSIDERED TO BE RELEVANT

| Category* | Citation of document, with indication, where appropriate, of the relevant passages | Relevant to claim No. |
| --- | --- | --- |
| Y | CN 103893891 A (BEIJING AEONMED CO., LTD.), 02 July 2014 (02.07.2014), description, paragraphs [0052]-[0060] and [0064]-[0070] | 1-7 |
| Y | CN 100998902 A (SHENZHEN MINDRAY BIO-MEDICAL ELECTRONICS CO., LTD.), 18 July 2007 (18.07.2007), description, page 2, paragraph 2 | 1-7 |
| A | CN 102114288 A (BEIJING AEONMED CO., LTD.), 06 July 2011 (06.07.2011), the whole document | 1-7 |
| A | CN 102397609 A (BEIJING AEROSPACE CHANGFENG CO., LTD.), 04 April 2012 (04.04.2012), the whole document | 1-7 |
| A | CN 104750129 A (BEIJING AEONMED CO., LTD.), 01 July 2015 (01.07.2015), the whole document | 1-7 |
| A | CN 104203093 A (KONINKLIJKE PHILIPS N.V.), 10 December 2014 (10.12.2014), the whole document | 1-7 |
| A | CN 102711889 A (ROYAL DUTCH PHILIPS ELECTRONICS LTD.), 03 October 2012 (03.10.2012), the whole document | 1-7 |

☒ Further documents are listed in the continuation of Box C.     ☒ See patent family annex.

| | |
| --- | --- |
| \*    Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
| --- | --- |
| 14 June 2016 (14.06.2016) | **28 June 2016 (28.06.2016)** |

| Name and mailing address of the ISA/CN: State Intellectual Property Office of the P. R. China No. 6, Xitucheng Road, Jimenqiao Haidian District, Beijing 100088, China Facsimile No.: (86-10) 62019451 | Authorized officer **HU, Yating** Telephone No.: (86-10) **52871091** |
| --- | --- |

Form PCT/ISA/210 (second sheet) (July 2009)

## INTERNATIONAL SEARCH REPORT

International application No.

**PCT/CN2016/079343**

**C (Continuation).** DOCUMENTS CONSIDERED TO BE RELEVANT

| Category* | Citation of document, with indication, where appropriate, of the relevant passages | Relevant to claim No. |
|---|---|---|
| A | CN 101785896 A (SHENZHEN PROBE SCIENCE & TECHNOLOGY CO., LTD.), 28 July 2010 (28.07.2010), the whole document | 1-7 |
| A | US 2003172929 A1 (MIELLNER, R.), 18 September 2003 (18.09.2003), the whole document | 1-7 |

Form PCT/ISA/210 (continuation of second sheet) (July 2009)

## INTERNATIONAL SEARCH REPORT
### Information on patent family members

International application No.

**PCT/CN2016/079343**

| Patent Documents referred in the Report | Publication Date | Patent Family | Publication Date |
|---|---|---|---|
| CN 103893891 A | 02 July 2014 | CN 103893891 B | 02 March 2016 |
| CN 100998902 A | 18 July 2007 | CN 100998902 B | 08 December 2010 |
| | | US 8196575 B2 | 12 June 2012 |
| | | US 2007163579 A1 | 19 July 2007 |
| CN 102114288 A | 06 July 2011 | CN 102114288 B | 23 October 2013 |
| CN 102397609 A | 04 April 2012 | None | |
| CN 104750129 A | 01 July 2015 | None | |
| CN 104203093 A | 10 December 2014 | WO 2013144925 A1 | 03 October 2013 |
| | | JP 2015512710 A | 30 April 2015 |
| | | EP 2830498 A1 | 04 February 2015 |
| | | US 2015059754 A1 | 05 March 2015 |
| CN 102711889 A | 03 October 2012 | JP 2013517827 A | 20 May 2013 |
| | | AU 2010343682 A1 | 13 September 2012 |
| | | US 2012298108 A1 | 29 November 2012 |
| | | CN 102711889 B | 03 June 2015 |
| | | JP 5775882 B2 | 09 September 2015 |
| | | WO 2011089491 A1 | 28 July 2011 |
| | | EP 2525859 A1 | 28 November 2012 |
| | | AU 2010343682 B2 | 29 January 2015 |
| CN 101785896 A | 28 July 2010 | CN 101785896 B | 02 November 2011 |
| US 2003172929 A1 | 18 September 2003 | WO 0143806 A2 | 21 June 2001 |
| | | EP 1239911 A2 | 18 September 2002 |
| | | DE 19961206 A1 | 05 July 2001 |

Form PCT/ISA/210 (patent family annex) (July 2009)